# EUROPÄISCHE PATENTANMELDUNG

(11) **EP 2 535 104 A1**
(43) Veröffentlichungstag der Anmeldung: **19.12.2012**
(21) Anmeldenummer: 12172310.0
(22) Anmeldetag: 15.06.2012
(51) Int. Cl.: B01F 7/00, C12M 1/02

(54) **Fermenter einer Biogasanlage und Misch- und Fördervorrichtung hierfür**

(30) Priorität: 15.06.2011 DE 102011051087
(71) Anmelder: Völkl, Robert, 95643 Tirschenreuth (DE)
(72) Erfinder: Völkl, Robert, 95643 Tirschenreuth (DE)
(74) Vertreter: Lang, Christian

(57) **Zusammenfassung**

Die vorliegende Erfindung betrifft eine Misch - und/oder Fördereinrichtung für einen Fermenter (1) mit mindestens einem Lager (6), mit welchem ein bewegliches, insbesondere drehbares Teil beweglich, insbesondere drehbar aufgenommen oder gelagert ist, wobei das Lager mindestens eine Lagerkomponente aus einem selbstschmierenden Kunststoff aufweist.

## Beschreibung

### HINTERGRUND DER ERFINDUNG

### GEBIET DER ERFINDUNG

Die vorliegende Erfindung betrifft einen Fermenter in einer Biogasanlage sowie eine Misch - und/oder Fördervorrichtung hierfür.

### STAND DER TECHNIK

In Biogasanlagen wird aus biologischen Materialien unter Einsatz von Bakterien Gas erzeugt, welches zu Heizzwecken oder zur elektrischen Energiegewinnung eingesetzt werden kann. Derartige Biogasanlagen weisen ein-, zwei- oder mehrstufige Fermenter auf, in denen die Biomasse von den eingesetzten Bakterien unter geeigneten Bedingungen, wie ausreichender Wärme und dergleichen, zu Biogas umgesetzt wird. Die Biomasse muss hierfür umgewälzt und in Bewegung gehalten werden, damit die Bakterien in Kontakt mit dem gesamten Material kommen, sodass das gesamte Material umgesetzt werden kann. Darüber hinaus kann sich auf der Biomasse eine Schwimmschicht bilden, die dazu führt, dass das entstehende Gas nicht aus der Biomasse aufsteigen kann. Auch diese Schwimmschichten müssen durch Rührwerke, Mischer oder Mixer zerstört werden und das oben aufschwimmende leichte Material muss in untere Bereiche der Biomasse befördert werden.

Entsprechend sind verschiedene Rührwerke oder Mischer bekannt, wie beispielsweise Tauchmotorrührwerke, Langwellenrührwerke, Stabmixer, Vertikal-Paddel-Rührwerke, Horizontal-Paddel-Rührwerke oder Haspelrührwerke. Allen diesen Rührwerken gemeinsam ist, dass sie drehende Bauteile, wie Wellen mit entsprechenden Propellern, Paddeln oder sonstigen Rotorschaufeln oder Fördereinrichtungen aufweisen, die entsprechend über die Welle gedreht werden. Die drehbaren Bauteile müssen in geeigneter Weise gelagert werden, wobei unter Umständen auch Lager innerhalb des Fermenters vorgesehen werden müssen, die zudem in Kontakt mit der Biomasse gelangen. Derartige Lager sind schwer zugänglich und der Aufwand für einen Austausch der Lager oder eine Wartung der Lager ist deshalb sehr hoch.

### OFFENBARUNG DER ERFINDUNG

### AUFGABE DER ERFINDUNG

Es ist deshalb Aufgabe der vorliegenden Erfindung dem oben beschriebenen Problem abzuhelfen und eine Verbesserung von Fermentern bzw. Misch - und/oder Fördereinrichtugen, insbesondere Rührwerken, Mischern oder Mixern für Biogasanlagen bereitzustellen, die den Aufwand für den Austausch oder die Wartung von Misch - und/oder Fördereinrichtugnen und deren Lagern in Fermentern vermindert.

### TECHNISCHE LÖSUNG

Diese Aufgabe wird gelöst mit einer Misch - und/oder Fördereinrichtung gemäß Anspruch 1 sowie einem Fermenter nach Anspruch 11. Vorteilhafte Ausgestaltungen sind Gegenstand der abhängigen Ansprüche.

Unter Lager wird bei der vorliegenden Erfindung jedes Bauteil einer Misch - und/oder Fördereinrichtung s verstanden, welches ein bewegliches Teil aufnimmt, so dass zwischen dem Lager und dem beweglichen Teil eine Relativbewegung ermöglicht wird. Die Relativbewegung schließt neben Rotationsbewegungen auch Linearbewegungen, beliebige Kurvenbewegungen und Kombinationen daraus ein. Insbesondere werden unter Lager auch Führungen oder Dichtungen gegenüber beweglichen Teilen verstanden. Entsprechend werden auch die Begriffe Lagerkomponenten und Lagerpartner, die nachfolgend Verwendung finden, in einem entsprechend breiten Umfang verwendet.

Unter Fermenter bzw. Fermenterbehälter wird bei der vorliegenden Erfindung jede Art von Bauteil bzw. Behälter verstanden, in dem Biomasse aufgenommen wird, welche durch ein Misch - und/oder Fördereinrichtung vermischt und/oder bewegt werden soll. Insbesondere werden auch Behälter einer Biogasanlage mit anderen Funktionen, wie Vorgärer, Nachgärer etc. darunter verstanden.

Obwohl in Rührwerken für Fermenter bereits Teflonlager eingesetzt werden, die bereits wartungsarm sind und eine hohe Lebensdauer gewährleisten, sodass lange Wartungs- oder Austauschintervalle für derartige Lager von Fermenter-Rührwerken möglich sind, wird nach der vorliegenden Erfindung vorgeschlagen, dies in der Weise weiter zu verbessern, dass selbstschmierende Kunststoffe als Lagerwerkstoffe für die Lager von Misch - und/oder Fördereinrichtungen insbesondere im Bereich des Fermenterinneren eingesetzt werden.

Beispielsweise wird bei einem Horizontal-Paddel-Rührwerk für einen Fermenter die Rührwerkswelle mit den daran angeordneten Paddeln üblicherweise in der Fermenteraußenwand sowie einem Gegenlager im Inneren des Fermenters gelagert. Die vorliegende Erfindung ist somit insbesondere, wenn auch nicht ausschließlich, auf das im Inneren des Fermenters am Gegenlager vorgesehene Lager gerichtet. Durch den Einsatz eines selbstschmierenden Kunststofflagers kann auf eine Wartung, bei der das Lager geschmiert werden muss, vollständig verzichtet werden, sodass die Wartungsintervalle entfallen. Außerdem wird durch das Vorsehen einer sich immer wieder erneuernden Schmierung des Lagers im Bereich des Fermenters die Lebensdauer des entsprechenden Lagers unter den dort herrschenden aggressiven Umgebungsbedingungen erhöht.

Der selbstschmierende Kunststoff kann durch einen Kunststoff gebildet werden, der Mikrokapseln enthält, die entsprechende Schmierstoffe aufweisen. Die Mikrokapseln werden durch Verschleiß zerstört, sodass das darin enthaltene Schmiermittel entweichen kann. Das Schmiermittel kann aus festen oder flüssigen Schmierstoffen, wie Ölen, Fetten oder dergleichen gebildet sein. Insbesondere können pflanzliche Öle oder Bioöle, wie Rapsöl oder dergleichen, zum Einsatz kommen.

Die Mikrokapseln können dem Kunststoff bei der Verarbeitung zugefügt werden, sodass der entsprechende Kunststoff mit Mikrokapseln beispielsweise durch Spritzguss verarbeitet werden kann und somit spritzgegossene Kunststofflager mit Selbstschmiereffekt bei den Misch - und/oder Fördereinrichtung en eingesetzt werden können.

Der Kunststoff, der die Mikrokapseln enthält, kann aus jedem geeigneten Kunststoff, welches für Lager eingesetzt werden kann, gebildet sein, insbesondere aus Polytetrafluorethylen (Teflon).

Das Lager kann mindestens zwei zueinander bewegliche Lagerpartner umfassen. Auch Lager mit mehreren Lagerkomponenten, insbesondere segmentierte Lager sind möglich.

Die zueinander beweglichen Lagerpartner können alle, insbesondere beide oder nur einer davon den selbstschmierenden Kunststoff aufweisen oder vollständig aus diesem gebildet sein. Beispielsweise können Lagerkomponenten eine Beschichtung aus dem selbstschmierenden Kunststoff aufweisen.

Die Lagerkomponenten bzw. das gesamte Lager können mittels Spritzguss hergestellt werden. Zudem kann das Lager gekapselt ausgeführt sein, um den Schmierstoff nicht entweichen zu lassen und/oder das Lager vor Umgebungseinflüssen zu schützen.

### KURZBESCHREIBUNG DER FIGUREN

Die beigefügten Zeichnungen zeigen in rein schematische Weise in
- Fig.1: einen Querschnitt durch einen Fermenterbehälter mit einer ersten Ausführungsform eines erfindungsgemäßen Rührwerks;
- Fig.2: einen Querschnitt durch einen Fermenterbehälter mit einer zweiten Ausführungsform eines erfindungsgemäßen Rührwerks;
- Fig.3: einen Querschnitt durch einen Fermenterbehälter mit einer dritten Ausführungsform eines erfindungsgemäßen Rührwerks;
- Fig. 4: einen Querschnitt durch einen Fermenterbehälter mit einer Ausführungsform eines erfindungsgemäßen Stabmixers;
- Fig. 5: eine perspektivische Darstellung eines erfindungsgemäßen Paddelrührwerks mit verstellbaren Paddeln;
- Fig. 6: eine Schnittansicht eines Teils einer erfindungsgemäßen Einbringungsvorrichtung mit einem Schneckenförder;

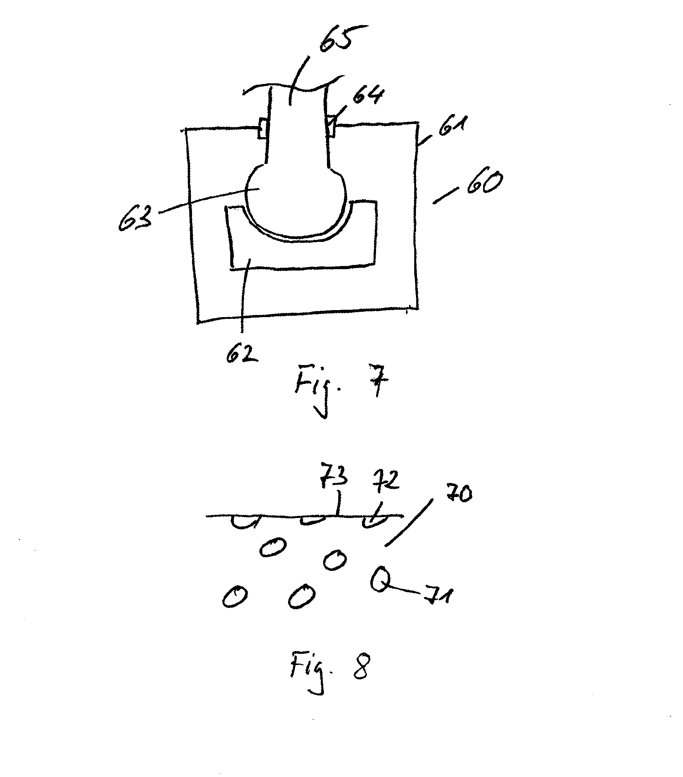

- Fig.7: eine Querschnittdarstellung einer Ausführungsform eines erfindungsgemäßen Lagers; und in
- Fig.8: einen teilweisen Querschnitt durch die Oberfläche einer erfindungsgemäßen Lagerkomponente.

### AUSFÜHRUNGSBEISPIELE

Weitere Vorteile, Kennzeichen und Merkmale der vorliegenden Erfindung werden bei der nachfolgenden detaillierten Beschreibung von Ausführungsbeispielen deutlich. Allerdings ist die Erfindung nicht auf diese Ausführungsbeispiele beschränkt.

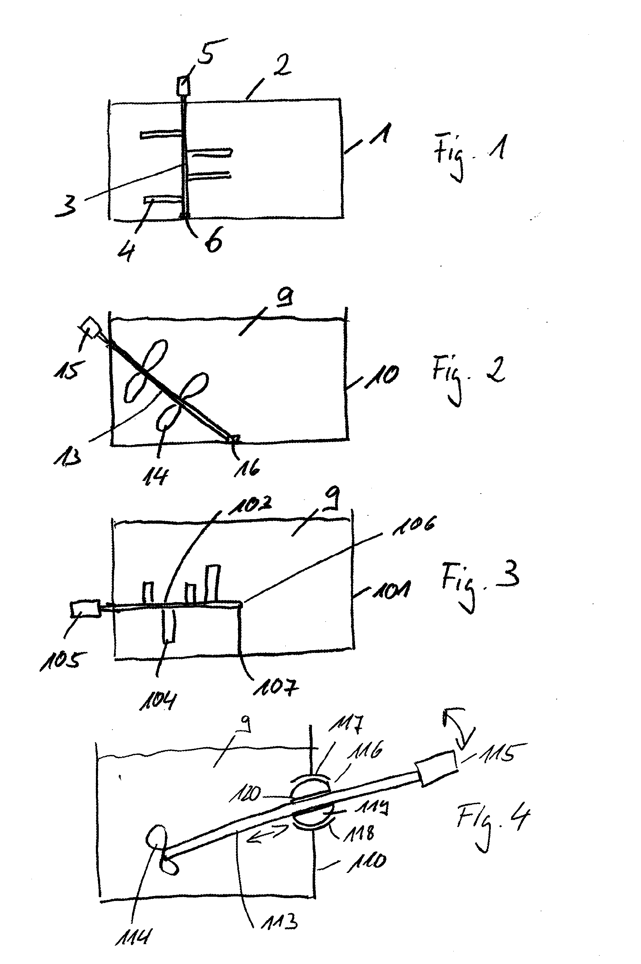

Figur 1 zeigt ein erstes Ausführungsbeispiel eines Rührwerks gemäß der vorliegenden Erfindung. Die Figur 1 zeigt einen Querschnitt durch einen Fermenterbehälter1 mit einem Deckel 2, durch den hindurch ein Rührwerk angeordnet und mit dem Lager 6 auf dem Boden des Fermenterbehälters 1 gelagert ist. Das Rührwerk umfasst einen Rührwerksstab 3 bzw. eine Welle, an der Paddel 4 angeordnet sind. Der Rührwerkstab 3 ist mit einem Motor 5 verbunden, der für den Antrieb des Rührwerkstabs 3 sorgt, sodass die Paddel 4, die am Rührwerksstab 3 angeordnet sind, sich drehend im Fermenterbehälter 1 bewegen. Bei einem derartigen Vertikal - Paddelrührwerk bewegen sich somit die Paddel 4 in horizontalen Ebenen und durchmischen die im Fermenterbehälter 1 befindliche Biomasse.

Die Figur 2 zeigt ein sogenanntes Langwellenrührwerk, welches einerseits in einer Seitenwand des Fermenterbehälters 10 gelagert ist und andererseits über ein Lager 16 am Boden des Fermenterbehälters 10 aufliegt. Das Rührwerk der Figur 2 umfasst wieder einen Rührwerksstab 13, an dem Paddel 14 angeordnet sind, sowie einen Motor 15, der den Rührwerksstab 13 drehend antreibt. Durch die drehende Bewegung der Paddel 14 wird auch hier Biomasse 9 im Fermenterbehälter 10 vermischt und in Bewegung gehalten.

Die Figur 3 zeigt ein sogenanntes Horizontal - Paddelrührwerk, bei welchem der Rührwerksstab 103 einerseits in einer Seitenwand des Fermenterbehälters 101 sowie auf einer Stütze 107 gelagert ist. Zwischen Stütze 107 und Rührwerkstab 103 ist wiederum ein Lager 106 vorgesehen. Entsprechende Lager bzw. Führungen oder Dichtungen können auch zwischen den Rührwekstäben 3, 13, 103 einerseits und dem Deckel 2 bzw. den Seitenwänden der Fermenterbehälter 10,101 vorgesehen sein.

Die Fig. 4 zeigt in einer weiteren Querschnittsansicht einen Behälter 110, in dem Biomasse 9 aufgenommen ist und der über ein Lager 116 in der Seitenwand einen Stabmixer aufgenommen hat, der sowohl in verschiedenste Richtungen verschwenkt werden kann als auch aus dem Behälter teilweise herausgezogen oder hineingeschoben werden kann.

Der Stabmixer weist einen Stab 113 auf, an dessen Ende ein Propeller 114 vorgesehen ist, der über den Motor 115 angetrieben werden kann. Wie die Doppelpfeile zeigen, kann der Stab 113 sowohl linear verschoben als auch verschwenkt werden. Das Lager 116 weist hierzu zwei Lagerschalen 117 und 118 auf, die eine Lagerkugel 119 einschließen. Innerhalb der Lagerschalen 117, 118, ist die Lagerkugel 119 in verschiedenen Richtungen drehbar aufgenommen.

Innerhalb der Kugel ist eine Durchführung 120 vorgesehen, durch die hindurch der Stab 113 geführt ist. In der Durchführung 120 ist eine Hülse aus selbstschmierendem Kunststoff vorgesehen, so dass der Stab 113 entlang dieser Hülse gleiten kann. Auch die Lagerschalen 117, 118 sind aus einem selbstschmierenden Kunststoff gebildet oder mit einem derartigen ausgelegt, so dass die Kugel 119 sich in den Lagerschalen 117, 118 bewegen kann. Alternativ oder zusätzlich könnte auch die Kugel 119 aus einem selbstschmierenden Kunststoff gebildet sein.

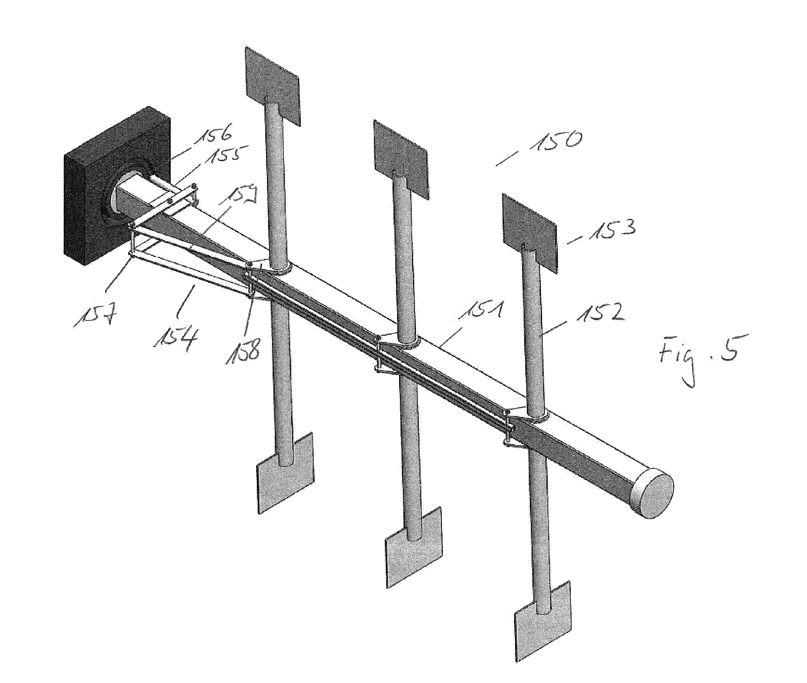

Die Fig. 5 zeigt ein Paddelrührwerk 150 mit verstellbaren Paddeln 153. Die Paddel 153 sind mit Paddelstäben 152 in einer Welle 151 drehbar aufgenommen, so dass sie sich um die Längsachse der Paddelstäbe 152 drehen können. Die Paddelstäbe 152 sind mit Hebeln 158 drehfest verbunden, welche wiederum über ein Gestänge 159 und entsprechende Gelenke 157 mit einem Führungsstab 155 verbunden sind, der in einer Kulissenführung 156 geführt ist.

Bei der Drehung der Welle 151 um ihre Längsachse wird der Führungsstab 155 in einer Nut der Kulissenführung 156 bewegt, deren Nutboden so ausgebildet ist, dass der Stab in Abhängigkeit von der Drehstellung der Welle 151 entlang der Längsrichtung der Welle 151 bewegt wird. Dadurch wird das Gestänge 159 mit den Gelenken 157 und somit der Kupplungsmechanismus 154 betätigt, so dass die Hebel 158 verschwenkt werden. Dadurch kann die Stellung der Paddel 153 bezüglich der Drehrichtung verändert werden. Beim Eintauchen der Paddel 153 in die Biomasse sind die Paddel mit ihren Paddelflächen quergestellt, so dass sie oben aufschwimmende Gegenstände in die Biomasse eindrücken. Sobald sie den tiefsten Punkt ihrer Drehbewegung erreicht haben, werden die Paddel, wie in Fig. 5 gezeigt ist, mit ihren Paddelflächen parallel zur Drehbewegung gestellt, so dass sie das nach unten gedrückte Biomassesubstrat nicht wieder an die Oberfläche befördern.

Auch bei einem derartigen Paddelrührwerk sind an verschiedensten Stellen selbstschmierende Kunststoffe eingesetzt, und zwar einerseits an den Gelenken 157, der drehbaren Lagerung der Paddelstäbe 152 sowie im Bereich der Kulissenführung 156, die einen selbstschmierenden Kunststoff aufweisen kann.

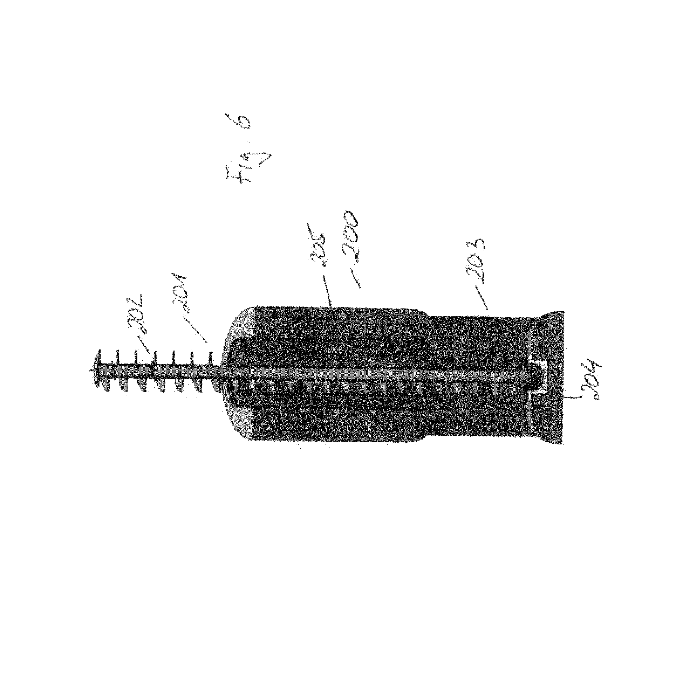

Die Fig. 6 zeigt in einem Querschnitt einen Teil einer Einbringungsvorrichtung als ein weiteres Anwendungsbeispiel für den Einsatz von selbstschmierenden Kunststoffen in einer Biogasanlage bzw. einem Fermenter. Die Fig. 6 zeigt eine Stütze mit einem in der Darstellung von oben nach unten verlaufenden Kanal 205, der durch die Stütz hindurch verläuft, so dass an einer oberen Öffnung 201 Biomasse eingeführt werden kann, die durch einen Schneckenförderer 202, der durch den Kanal 205 verläuft, nach unten befördert wird. Im unteren Bereich der Stütze ist quer zum Kanal 205 eine Durchgangsöffnung 203 vorgesehen, so dass das von der Schnecke 202 nach unten beförderte Material beidseits der Schnecke in den Fermenter austreten kann. Die Förderschnecke 202 ist in einem Lager 204 gelagert, wobei die beiden Lagerkomponenten, nämlich einerseits der Kugelkopf des Schneckenförderers 202 und andererseits die Lagerschale aus einem selbstschmierenden Kunststoff gebildet sein können.

Die Figur 4 zeigt ein vereinfachtes schematisches Beispiel eines möglichen Lagers 60, das lediglich dazu dient, das Prinzip zu erläutern. Bei dem Lager 60 handelt es sich um ein gekapseltes Lager, welches eine Kapselung 61 in Form eines Gehäuses aufweist, in dem die Lagerpartner 62,63, die sich gegeneinander bewegen, von der Umgebung abgetrennt aufgenommen sind. Die Lagerpartner 62,63 werden im gezeigten Ausführungsbeispiel der Figur 4 durch eine Lagerpfanne 62 und einen Wellenkopf 63 gebildet, der in der Lagerpfanne 62 aufgenommen und in dieser drehbar gelagert ist. Sowohl die Lagerpfanne 62, als auch der Wellenkopf 63, der mit einer Rührwerkswelle 65 verbunden ist, können aus einem selbstschmierenden Kunststoff gebildet sein. Alternativ kann auch lediglich einer der Lagerpartner, nämlich die Lagerpfanne 62 oder der Wellenkopf 63 aus dem selbstschmierenden Kunststoff gebildet sein oder den selbstschmierenden Kunststoff umfassen. Beispielsweise könnte der selbstschmierende Kunststoff als Beschichtung im Bereich der Lageroberflächen der Lagerpfanne 62 und/oder des Wellenkopfes 63 angeordnet sein. Ganz allgemein gilt für die erfindungsgemäßen Rührwerke und die darin verwirklichten Lager, Führungen, Dichtungen und dergleichen, die mit selbstschmierenden Kunststoffen verwirklicht sind oder diese umfassen, dass der selbstschmierende Kunststoff auch als Teil einer Beschichtung aufgebracht sein kann.

Da die Lagerpartner 62,63 insbesondere aus hochbeständigen Kunststoffen gebildet sein können oder diese umfassen können, wie beispielsweise Polytetrafluorethylen (PTFE), können bei Lagern für Rührwerke von Fermentern, Nachgärern oder vergleichbaren Behältern von Biogasanlagen auch Lager eingesetzt werden, die keine Kapselung 61 umfassen, sodass die Lagerpartner 62,63 unmittelbar in Kontakt mit der Biomasse sind.

Bei einer Kapselung 61, wie sie in der Ausführungsform der Figur 4 dargestellt ist, ist die Welle 65 bzw. der Rührwerksstab durch eine Öffnung der Kapselung 61 hindurchgeführt, wobei eine Dichtung 64 vorgesehen ist, die ebenfalls aus einem selbstschmierenden Kunststoff gemäß der Erfindung ausgebildet sein kann.

Darüber hinaus ist es auch möglich, Lager mit anderen selbstschmierenden Lagerwerkstoffen oder mit entsprechend in die Kapselung 61 eingefüllten Schmierstoffen zu verwenden, wobei dann wiederum für die Dichtung der Kapselung 61 gegenüber der Welle 65 ein selbstschmierender Kunststoff gemäß der Erfindung vorgesehen sein kann.

Die Figur 5 zeigt in einer schematischen Weise den Aufbau und die Wirkungsweise eines selbstschmierenden Kunststoffs gemäß der Erfindung. Figur 5 zeigt einen teilweisen Querschnitt durch eine Oberfläche 73, die beispielsweise als Lageroberfläche für die Lagerpfanne 62 oder dem Wellenkopf 63 dienen kann. In dem Kunststoffmaterial 70, welches jedes geeignete Kunststoffmaterial, wie beispielsweise Polytetrafluorethylen sein kann, sind Kapseln 71 eingelagert, die an der Oberfläche 72 durch Verschleiß und Reibung geöffnet sind, sodass in den Kapseln 71 enthaltene Schmierstoffe entweichen können. Die Schmierstoffe können hierbei als Festschmierstoffe ausgebildet sein, wie beispielsweise Molybdändisulfid oder Graphit oder auch als flüssige oder viskose Schmierstoffe, wie Fette, Öle und dergleichen.

Bei einem Abtrag des Kunststoffs 70 und dadurch Absenken der Oberfläche 73 werden immer wieder neue Kapsel 71 angeschnitten, sodass immer wieder neuer Schmierstoff entweichen und für die Schmierung an der Oberfläche 73 zur Verfügung stehen kann.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Änderungen in der Weise möglich sind, dass einzelne Merkmale weggelassen oder andersartige Kombinationen von Merkmalen verwirklicht werden, sofern der Schutzbereich der beigefügten Ansprüche nicht verlassen wird. Insbesondere ist es für den Fachmann selbstverständlich, dass unterschiedlichste Rührwerke und Lager mit entsprechenden Lagerkomponenten aus selbstschmierendem Kunststoff verwirklicht werden können. Die vorliegende Offenbarung umfasst sämtliche Kombinationen aller vorgestellter Einzelmerkmale.

## Patentansprüche

1. Misch - und/oder Fördereinrichtung für einen Fermenter mit mindestens einem Lager (6,16,106), mit welchem ein bewegliches, insbesondere drehbares Teil beweglich, insbesondere drehbar aufgenommen oder gelagert ist,
**dadurch gekennzeichnet, dass**
das Lager mindestens eine Lagerkomponente (62,63,64) einen selbstschmierenden Kunststoff aufweist.

2. Misch - und/oder Fördereinrichtung nach Anspruch 1,
**dadurch gekennzeichnet, dass**
das Lager mindestens zwei Lagerpartner (62,63) als Lagerkomponenten umfasst, die relativ gegeneinander beweglich sind, wobei mindestens ein Lagerpartner oder beide gegeneinander beweglichen Lagerpartner zumindest teilweise oder vollständig aus einem selbstschmierenden Kunststoff gebildet sind.

3. Misch - und/oder Fördereinrichtung nach einem der vorhergehenden Ansprüche, **dadurch gekennzeichnet, dass**
der selbstschmierende Kunststoff Mikrokapseln (71) mit Schmierstoff enthält.

4. Misch - und/oder Fördereinrichtung nach Anspruch 3,
**dadurch gekennzeichnet, dass**
der Schmierstoff fest oder flüssig ist.

5. Misch - und/oder Fördereinrichtung nach Anspruch 3,
**dadurch gekennzeichnet, dass**
der Schmierstoff ein Bioöl ist oder dieses umfasst.

6. Misch - und/oder Fördereinrichtung nach einem der vorhergehenden Ansprüche,
**dadurch gekennzeichnet, dass**
der Kunststoff Polytetrafluorethylen umfasst.

7. Misch - und/oder Fördereinrichtung nach einem der vorhergehenden Ansprüche,
**dadurch gekennzeichnet, dass**
die mindestens eine Lagerkomponente oder das Lager ganz oder teilweise durch Spritzguss hergestellt sind.

8. Misch - und/oder Fördereinrichtung nach einem der vorhergehenden Ansprüche,
**dadurch gekennzeichnet, dass**
das Lager gekapselt oder segmentiert ist.

9. Misch - und/oder Fördereinrichtung nach einem der vorhergehenden Ansprüche,
**dadurch gekennzeichnet, dass**
die Lagerkomponente aus selbstschmierendem Kunststoff eine Dichtung (64) oder eine Führung ist.

10. Misch - und/oder Fördereinrichtung nach einem der vorhergehenden Ansprüche,
**dadurch gekennzeichnet, dass**
die Misch - oder Fördereinrichtung ein Rührwerk, Tauchmotorrührwerk, Langwellenrührwerk, Paddel-Rührwerk, Paddel-Rührwerk mit verstellbaren Paddeln, Vertikal-PaddelRührwerk, Horizontal-Paddel-Rührwerk, Haspelrührwerk, Mischer, Schwerkraft-Mischer, Mixer , Stabmixer, ein Schneckenförderer oder eine Eintragsschnecke ist.

11. Fermenter für eine Biogasanlage mit einem Fermenterbehälter (1,10,101) und einer darin oder daran angeordneten Misch - und/oder Fördereinrichtung nach einem der vorhergehenden Ansprüche.

12. Fermenter nach Anspruch 11,
**dadurch gekennzeichnet, dass**
das Misch - und/oder Fördereinrichtung mindestens ein im Fermenter befindliches Lager (6,16,106) aufweist, welches mindestens eine Lagerkomponente umfasst, die aus einem selbstschmierenden Kunststoff gebildet ist.
